# EUROPÄISCHE PATENTANMELDUNG

(11) **EP 0 627 204 A2**
(43) Veröffentlichungstag der Anmeldung: **07.12.1994**
(21) Anmeldenummer: 94107417.1
(22) Anmeldetag: 13.05.1994
(51) Int. Cl.: A61F 2/44, A61F 2/30

(54) **Implantat zum Versteifen benachbarter Wirbelknochen**

(30) Priorität: 04.06.1993 DE 4318700
(71) Anmelder: ESKA Implants GmbH, 23556 Lübeck (DE)
(72) Erfinder: Strohfeld, Gerhard, Dr., D-30938 Grossburgwedel (DE); Springer, Hans-Herbert, Dr., D-19055 Schwerin (DE)
(74) Vertreter: Fuchs, Luderschmidt & Partner Patentanwälte

(57) **Zusammenfassung**

Es wird ein Implantat zum Versteifen benachbarter Wirbelknochen angegeben, welches eine Scheibe (2) aus einer offenporigen oder offenzelligen Metallstruktur und mindestens einen durch die Scheibe (2) hindurchlaufenden Durchbruch (8) enthält.

## Beschreibung

Die Erfindung betrifft ein scheibenförmiges Implantat zum Versteifen benachbarter Wirbelknochen.

Derartige Implantate sind in verschiedener Ausgestaltung bekannt und dienen dazu, benachbarte Wirbelknochen fest miteinander zu verbinden, um die Relativbewegung der benachbarten Wirbelknochen bei geschädigter oder abgetragener Bandscheibe zu verhindern.

Aus der EP-A 0 271 501 ist ein derartiges Implantat bekannt, welches aus einem zylinderförmigen Block mit offenzelliger oder offenporiger Oberflächenstruktur besteht und in eine Ausnehmung implantiert wird, die an den sich gegenüberliegenden Knochenflächen benachbarter Wirbelkörper der Wirbelsäule resiziert wird. Dabei wird der für das Implantat notwendige Aufnahmeraum zwischen benachbarten Wirbelkörpern näherungsweise der Form des Implantates angepaßt, um einen formschlüssigen Verbund zwischen den Wirbelknochen zu erzielen, wobei das angrenzende Knochengewebe in die offenporige Umfangsfläche des Implantates einwachsen, und somit die benachbarten Wirbelkörper drehfest miteinander verbinden soll. Nachteilig ist es dabei, daß auch dann ein relativ großes Knochenvolumen resiziert werden muß, wenn die einander Zugekehrten, tragenden Flächen der benachbarten Wirbelkörper keine oder nur geringe Verschleißerscheinungen aufweisen.

Aufgabe der Erfindung ist es, ein Implantat der eingangs genannten Art anzugeben, das eine wirksame Versteifung benachbarter Wirbelkörper bei knochenschonender Implantation ermöglicht.

Diese Aufgabe wird bei dem Implantat der eingangs genannten Art erfindungsgemäß gelöst durch eine Scheibe aus einer offenzelligen oder offenporigen Metallstruktur, durch die mindestens ein großflächiger Durchbruch hindurchverläuft.

Die Vorteile der Erfindung liegen insbesondere darin, daß das scheibenförmige Implantat etwa die Form einer natürlichen Bandscheibe besitzt und nach Resektion der natürlichen Bandscheibe zwischen die benachbarten Wirbelkörper eingeschoben werden kann, und daß der oder die großflächigen Durchbrüche dabei mit Partikeln aus Spongiosagewebe gefüllt werden, welches an die Grenzflächen der Wirbelkörper anwächst und außerdem in die offenporige Metallstruktur hineinwächst. Dadurch findet - durch das Implantat hindurch - eine Verwachsung und damit Versteifung der benachbarten Wirbelkörper statt, und außerdem verwächst das im Durchbruch zusammenwachsende Spongiosagewebe noch mit der offenporigen Metallstruktur, so daß nach einer Einwachsphase ein stabiler Verbund aus Wirbelkörpern und Implantat realisiert wird.

Besonders bevorzugt werden durch die Scheibe hindurch zwei Durchbrüche eingearbeitet, die bevorzugt einen kreiszylindrischen Querschnitt aufweisen. Der Radius der kreiszylindrischen Durchbrüche ist dabei so bemessen, daß zwischen den Durchbrüchen nur eine dünne Trennwand verbleibt, und daß auch die Durchbrüche einen möglichst großen Querschnitt aufweisen, um einen möglichst großen Aufnahmeraum für Spongiosagewebe zur Verfügung zu stellen. Wesentlich ist dabei, daß die Dicke der Scheibe näherungsweise der Dicke einer zu ersetzenden natürlichen Bandscheibe entspricht, daß also der Innenraum der Durchbrüche nicht durch eine Vergrößerung der Scheibendicke vergrößert werden kann. Bei zwei eingearbeiteten Durchbrüchen, die bevorzugt einen kreiszylindrischen Querschnitt aufweisen, läßt sich eine großflächige Verwachsung der von dem Implantat beabstandet gehaltenen Wirbelkörpern erzielen. Alternativ ist eine großflächige Verwachsung der vom Implantat auf Distanz gehaltenen Wirbelkörper auch durch einen Durchbruch realisierbar, der bevorzugt einen Langloch-Querschnitt besitzt.

Besonders bevorzugt ist am Umfang der Scheibe mindestens ein Steg vorgesehen, der zum Beispiel endlos am Umfang umläuft und - in einer weiteren bevorzugten Ausführungsform der Erfindung - die offenporige Metallstruktur mindestens an einer oder an beiden gegenüberliegenden Oberflächen der Scheibe überragt. Der Steg läßt sich mit Spitzen versehen oder schneidenförmig ausbilden, damit unter dem Andruck der beiden anliegenden Wirbelkörper mit seinen Spitzen oder Schneiden ein vorgegebenes Maß in die Anlageflächen der Wirbelkörper hineindrückt und unter diesem Andruck eine erwünschte formschlüssige Primärkupplung der benachbarten Wirbelkörper realisiert.

Die Scheibe weist bevorzugt eine nierenförmige Umfangskontur auf, die dem Zwischenraum zwischen den benachbarten Wirbelkörpern näherungsweise entspricht.

Gemäß einer weiteren vorteilhaften Ausführungsform der Erfindung besitzt die Scheibe zwei konvex gekrümmte oder schräg geneigte Oberfläche, welche den konkaven Krümmungen der angrenzenden Wirbelkörper-Flächen angepaßt ist oder eine Positionskorrektur von benachbarten Wirbelkörpern ermöglichen.

Die Scheibe kann mindestens abschnittweise mit einem innenliegenden Kern aus massivem Material versehen werden, um die Eigenstabilität des Implantates zu erhöhen.

In einer besonders bevorzugten Ausführungsform sind die Durchbrüche nur von je einem ringförmigen Rand aus einer offenporigen oder offenzelligen Metallstruktur begrenzt, und diese ringförmigen Ränder sind mindestens in einem gemeinsamen Randabschnitt miteinander verbunden. Zusätzlich kann mindestens ein weiterer Verbindungsbügel in der Scheibenebene vorgesehen werden, der eine Brücke zwischen den beiden ringförmigen Rändern bildet.

Vorteilhafte Weiterbildungen der Erfindung sind durch die Merkmale der Unteransprüche gekennzeichnet.

Im folgenden wird ein Ausführungsbeispiel der Erfindung anhand der Zeichnungen näher erläutert. Es zeigen:
- Fig. 1: eine Aufsicht auf ein erfindungsgemäßes Implantat;
- Fig. 2: eine Seitenansicht durch das Implantat der Fig. 1; und
- Fig. 3: eine weitere Ausführungsform des Implantats.

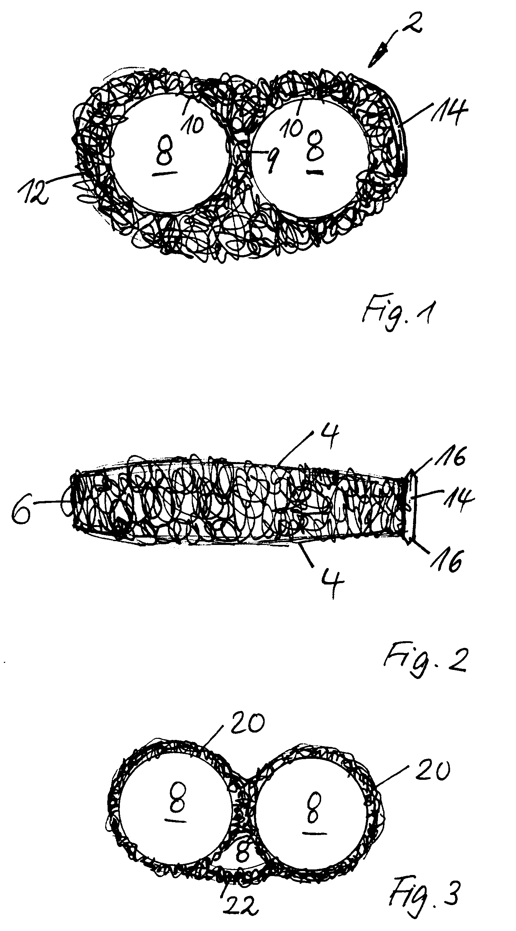

In den Fig. 1 und 2 ist ein Implantat 2 dargestellt, welches nach Revision der betreffenden Bandscheibe zur Versteifung der benachbarten Wirbelkörper dient. Das Implantat besitzt die Form einer Scheibe 2, die durchgängig aus einer offenporigen oder offenzelligen Metallstruktur hergestellt ist und durch zwei einander gegenüberliegende Oberflächen 4 und eine Umfangsfläche 6 begrenzt ist. Die Oberflächen 4 sind - in der dargestellten Ausführungsform - geringfügig konvex gekrümmt.

Die Scheibe 2 enthält zwei kreiszylindrische Bohrungen oder Durchbrüche 8, die mittels einer vergleichsweise dünnen Trennwand 9 voneinander getrennt sind und einen merklichen Flächenanteil der Scheibe 2 einnehmen. Der Durchmesser der Durchbrüche 8 ist so gewählt, daß zwischen den beiden Durchbrüchen 8 und an den am weitesten voneinander beabstandeten Abschnitten der Innenfläche 10 der Durchbrüche 8 eine vergleichsweise schmale Randzone 12 der Scheibe 2 verbleibt. Bei dieser Dimensionierung wird - bei im wesentlichen vorgegebener Scheibendicke - ein möglichst großer Innenraum der Durchbrüche 8 verwirklicht, der beim Implantieren der Scheibe mit zuvor anderweitig gewonnenem natürlichen Spongiosagewebe angefüllt wird.

Die Scheibe 2 ist nierenförmig ausgebildet, sie besitzt in der dargestellten Ausführungsform an ihrem Umfang 6 einen Steg 14, der sich über einen vorgegebenen Längenabschnitt erstreckt, und in einer alternativen, nicht dargestellten Ausführungsform endlos am Umfang umläuft. Der Steg 14 überragt an beiden Oberflächen 4 die offenporige Metall struktur geringfügig und läuft an seinen freien Enden 16 schneidenförmig oder dornenförmig aus.

Fig. 3 zeigt eine weitere Ausführungsform des Implantats, bei der die Durchbrüche 8 nur von ringförmigen Rändern 20 aus offenporigem oder offenzelligem metallischen Material begrenzt sind. Die Ränder 20 liegen in der Scheibenebene und sind zusätzlich mit einem Verbindungsbügel 22 miteinander gekoppelt, so daß zwischen den Rändern 20 und den Verbindungsbügeln 22 ein dritter, etwa keilförmiger Durchbruch 8 entsteht.

## Patentansprüche

1. Implantat zum Versteifen benachbarter
Wirbelknochen,
gekennzeichnet durch eine Scheibe (2) aus einer offenporigen oder offenzelligen Metallstruktur und mindestens einen Durchbruch (8) durch die Scheibe (2).

2. Implantat nach Anspruch 1,
gekennzeichnet durch mindestens zwei durch die Scheibe (2) hindurchlaufende Durchbrüche (8).

3. Implantat nach einem der vorstehenden Ansprüche,
dadurch gekennzeichnet, daß die Durchbrüche (8) einen kreiszylindrischen Querschnitt aufweisen.

4. Implantat nach einem der vorstehenden Ansprüche,
gekennzeichnet durch mindestens einen Steg (14) aus massivem Metall am Umfang der Scheibe (2).

5. Implantat nach Anspruch 4,
dadurch gekennzeichnet, daß der Steg (14) am Umfang endlos umläuft.

6. Implantat nach Anspruch 4 oder 5,
dadurch gekennzeichnet, daß der Steg (14) die offenporige Metallstruktur an mindestens einer Oberfläche (4) der Scheibe (2) überragt.

7. Implantat nach Anspruch 6,
dadurch gekennzeichnet, daß der Steg (14) die Oberfläche (4) der Scheibe (2) schneidenförmig überragt.

8. Implantat nach einem der vorstehenden Ansprüche, dadurch gekennzeichnet, daß die Scheibe (2) eine nierenförmige Umfangskontur aufweist.

9. Implantat nach einem der vorstehenden Ansprüche,
dadurch gekennzeichnet, daß die Scheibe (2) konvex gekrümmte oder schräg geneigte Oberflächen (4) besitzt.

10. Implantat nach einem der vorstehenden Ansprüche,
dadurch gekennzeichnet, daß die Durchbrüche (8) mittels einer dünnen Wand (9) voneinander getrennt sind.

11. Implantat nach einem der vorstehenden Ansprüche,
dadurch gekennzeichnet, daß an der Innenwand der Durchbrüche (8) je ein Steg aus massivem Metall umläuft.

12. Implantat nach einem der Ansprüche 1 bis 11,
dadurch gekennzeichnet, daß die Scheibe (2) mindestens abschnittweise einen innenliegenden Kern aus massivem Material enthält.

13. Implantat nach einem der Ansprüche 1 bis 12,
dadurch gekennzeichnet, daß die Durchbrüche (8) nur durch je einen ringförmigen Rand aus offenporiger oder offenzelliger Metallstruktur begrenzt sind, und daß die ringförmigen Ränder der Durchbrüche (8) miteinander verbunden sind.

14. Implantat nach Anspruch 13,
gekennzeichnet durch einen Verbindungsbügel zwischen den ringförmigen Rändern der Durchbrüche (8).
